# EUROPEAN PATENT SPECIFICATION

(11) **EP 3 793 469 B1**
(45) Date of publication and mention of the grant of the patent: **03.06.2026**
(21) Application number: 19728542.2
(22) Date of filing: 17.05.2019
(51) Int. Cl.: A61B 34/37, A61B 34/00, A61B 8/00

(54) **MASTER CONTROLLER ASSEMBLY FOR A ROBOTIC SURGERY SYSTEM, PARTICULARLY FOR MICROSURGERY**
MASTER-STEUERUNGSANORDNUNG FÜR EIN ROBOTISCHES CHIRURGIESYSTEM, INSBESONDERE FÜR DIE MIKROCHIRURGIE
ENSEMBLE CONTRÔLEUR MAÎTRE POUR SYSTÈME CHIRURGICAL ROBOTISÉ, PARTICULIÈREMENT POUR LA MICROCHIRURGIE

(30) Priority: 17.05.2018 IT 201800005469; 24.08.2018 IT 201800008179; 30.08.2018 IT 201800008251
(43) Date of publication of application: 24.03.2021
(73) Proprietor: Medical Microinstruments, Inc., Wilmington, DE 19801 (US)
(72) Inventor: SIMI, Massimiliano, 56121 Pisa (IT); PRISCO, Giuseppe Maria, 56121 Pisa (IT)
(74) Representative: Crippa, Paolo Ernesto
(86) International application number: PCT/IB2019/054095
(87) International publication number: WO 2019/220407

(56) References cited:
- WO-A1-01/41052
- DE-A1- 102010 009 065
- DE-A1- 102014 006 264
- US-A- 6 063 095
- US-A1- 2010 013 766
- US-A1- 2013 035 697
- US-B1- 6 594 552

## Description

### . Field of the invention

**.** It is an object of the present invention a master controller assembly for a robotic surgery system.

**.** Moreover, the present invention relates to a robotic surgery system.

**.** In particular, said robotic surgery system is suitable for microsurgery.

### . Background

**.** Robotic surgical assemblies comprising a master interface and a slave surgical tool are generally known in the field. Specifically, robotic surgical assemblies of the known type comprise a master control station able to control the motion of a slave surgical end-effector, as shown for example in document US-5876325**.** This document disclose a non-portable, robot-hung articulated appendices, which are hung to a beam fixedly constrained to the master control station, said appendices comprise master tools to control the slave surgical end-effector operating on a patient anatomy.

**.** Similar non-portable robot-held master tool solutions are shown, for example, in documents US-6063095**,** US-6424885 and US-6594552**,** wherein the appendix of the master control station acting as master tool to control the slave surgical end-effector comprises an appendix body rigidly constrained to the master control station. The transmission of motion to the slave end-effector is based on the detection of mechanical stress induced by urging the appendix body of the master control station in various spatial directions. Such an appendix body can be associated to a pair of opposite fins, each of said fins being constrained on one of its end to the appendix body in such way to form a cantilevered fins, suitable for receiving a manual command directed to activate the grip degree-of-freedom of the slave end-effector.

**.** However, non-portable, robot-hung or robot-held master tool solutions of the types described above exhibit some drawbacks. The provision of such control appendix mechanically constrained to the master control station of the robotic surgical assembly strongly limits the natural freedom of motion of the surgeon during surgery and forces the surgeon to operate in a predefined location from which the master control station, and particularly the control appendix attached thereto, is easily reachable. The discomfort for the surgeon is still enhanced due to the inability to real-time adjust during surgery the location, for example in terms of height from the soil, of such an appendix. That leads the surgeon to an untimely tiredness during surgery and to early focus loss.

**.** Often, surgeons have been trained for years to properly handle surgical tools suitable for operating directly on a patient anatomy. Surgical tools are generally portable tools and comprise a tool handle, suitable to be hand-held and manipulated by the surgeon, said handle being mechanically directly connected to a tool tip, suitable for operating on a patient anatomy. Some examples of traditional ophthalmic surgery surgical tools are shown in documents US-5634918 and WO-2012-064361**.** Such traditional surgery tools make the surgeon sure to know when the tool tip is free from touching the patient anatomy, in this way allowing the surgeon to safely (i.e. without transmitting actions on the patient anatomy) roll the tool handle between the fingers around the longitudinal axis of the tool handle, a gestural stress-reducing need rather common among surgeons for example useful for relax the hand muscles during surgery and to prevent muscular spasms.

**.** Robotic microsurgery, instead, forces the surgeon to use master tools to control the motion of an associated slave end-effector operating on a patient anatomy, and usually said master tools limit the comfort of the surgeon during surgery and often force the surgeon to an additional period of training for properly using the master tool to control the slave end-effector. The additional training period can be even lengthen if the of the shape and functionalities of the master tool are alien from a traditional surgical tool.

**.** Wearable master tool have been provided, as disclosed for example in document US-8996173**,** wherein a pair of rings are designed to be fit on surgeon's finger and wired to the robotic slave assembly. A codified gesture set of the surgeon's finger triggers a predefined slave end-effector action on the patient anatomy. Obviously, this solution requires a very long training to the surgeon for properly managing such a wearable master rings, in order to avoid to transmit unintended commands to the slave end-effector. Unintended command transmission to the slave should be avoided for patient safety reasons. Also documents DE-102014006264 and DE-102010009065 shows a wearable master tool.

**.** To overcome the deficiencies of known solution described above and in order to provide an hand-held manipulandum (i.e. from the Latin: "something to be manipulated") master tool having a shape which is familiar for most surgeons, documents WO-2017-064303 and WO-2017-064306**,** in the name of the same Applicant, show a master tool device which substantially replicates the appearance of a traditional surgery tweezers. Such master tool device comprises a pair of flexible strips of metal welded together in one of their ends to form a tweezers-like master input device. Suitably located sensors help the magnetic pad to track the motion of the tweezers and detect when the tweezers close, in order to mimic an object grasp and to transmit the detected motion to the slave surgical end-effector.

**.** Although satisfactory to improve the surgeon's comfort during surgery, this type of solution is prone to drawbacks. In particular, such flexible metal strips forming a tweezers-like device force a non-linear motion of the sensors placed on the free end of the metal strips, thus the detection of the manually-induced tweezers closing motion, for mimicking a sort of object grasp, often leads to measurement uncertainity and low sensing resolution. Mechanical vibrations arising in each metal strip during its elastic bending generate noise detected by the tracking pad. That could result in an unsatisfactory motion response of the slave end-effector that could even lead to serious complications in the patient body after surgery. Moreover, the tracking pad is suitable for generating a tracking magnetic field only from one side of the pad, forcing the surgeon not to move the manipulandum hand-held master tool on the back side of the pad, where the motion cannot be tracked, thus a command signal cannot be transmitted to the end-effector.

**.** Furthermore, documents US-2013-0035697**,** WO-2014-151621 and US-2015-038981 disclose a portable, hand-held master input tool manipulatable by a surgeon while moving in various locations of the operating arena. This solution exploits video-camera tracking of suitably designed balls that protrudes cantilevered from the portable hand-held master tool body. In other words, a set of three non-symmetric balls mounted on the master tool can be tracked by a camera apparatus provided on-robot to determine the position and orientation of the master input tool with the aim to transmit a command signal to the slave surgical end-effector.

**.** Although satisfactory under some points of view, this solution is prone to drawbacks. As the visual-cues-based tracking system allows the surgeon to operate while moving in various locations of the operating arena, at the same time force the robot to have a powerful control system and can result in an unwanted delay of transmission of motion to the slave end-effector, resulting in a discomfort for the surgeon.

**.** The need is felt to provide a master tool solution for robotic surgery able to overcome the drawbacks cited with reference to the prior art.

**.** The need is felt to provide a master tool for robotic surgery suitable for improving the surgeon's comfort and at the same time able to provide a high sensing accuracy.

**.** The need is felt to provide a master tool for robotic surgery able to reduce to a minimum the length of the surgeon training.

**.** The need is felt to provide a master tool for robotic surgery suitable for avoiding the transmission of unwanted command signal to the slave end-effector.

**.** The need is felt to provide a master tool for robotic surgery devoid of mechanical constraint to the master control station or to the slave robot.

### . Solution

**.** It is a scope of the present invention to overcome the drawbacks mentioned with reference to the prior art.

**.** These and other scopes are achieved by a master controller assembly according to claim 1, as well as a robotic surgery system according to claim 18.

**.** Some preferred embodiments are the subject of dependent claims.

**.** According to an aspect of the invention, a master controller assembly comprises at least one master input tool and at least one sensing assembly, wherein said master input tool comprising at least one manipulandum surface, designed to be hand-held by the surgeon's fingers and is mechanically unconstrained from said slave robot assembly, in such way that said master input tool being naturally movable, rotatable and spinnable by a surgeon. Said at least one manipulandum surface is a convex surface, so that said master input tool (106) can be rolled between surgeon's fingers around a tool longitudinal axis. Thereby the surgeon comfort during surgery is preserved. Said master input tool comprises a first elongated element having an first element elongated body, wherein said first element elongated body is a rigid body, and a second elongated element having an second element elongated body, wherein said second element elongated body is a rigid body, and a tool joint connecting and articulating said first element elongated body and said second element elongated body, providing a single degree of freedom of motion between said first element elongated body and said second element elongated body.

**.** The at least one sensing assembly detects at least the mutual position of said first element elongated body and said second element elongated body so that a gripping pressure action exerted by the surgeon's fingers on said master input tool moving said first element elongated body and said second element elongated body close one another other determines a paired grip motion of said surgical grip device.

**.** Said sensing assembly may comprise a pair of sensors received in respective slots of the master input tool body. Each slot may be mechanically shaped to be compatible with only one of the sensor.

**.** Each sensor of the sensing assembly may be encapsulated by a sterile barrier, such as a plastic bag or box, to allow the re-use of the sensors. The master input tool body may be disposable.

### . Figures

**.** Further characteristics and advantages of the master controller assembly and of the robotic surgery system according to the invention will appear from the description reported below of preferred embodiments, which are given as examples and are not meant to be limiting, which makes reference to the attached drawings, in which:

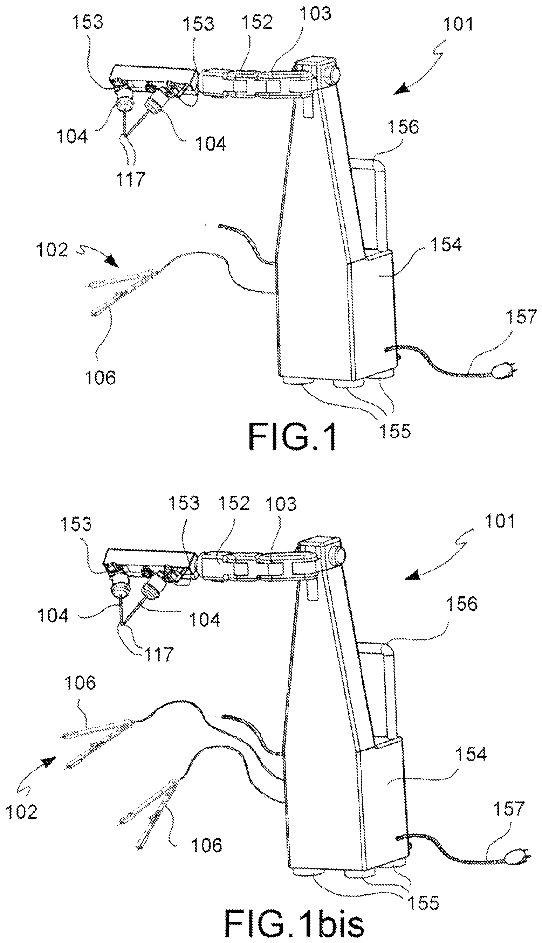

- figures 1 and This are perspective views showing a robotic surgery system, according to some embodiments;

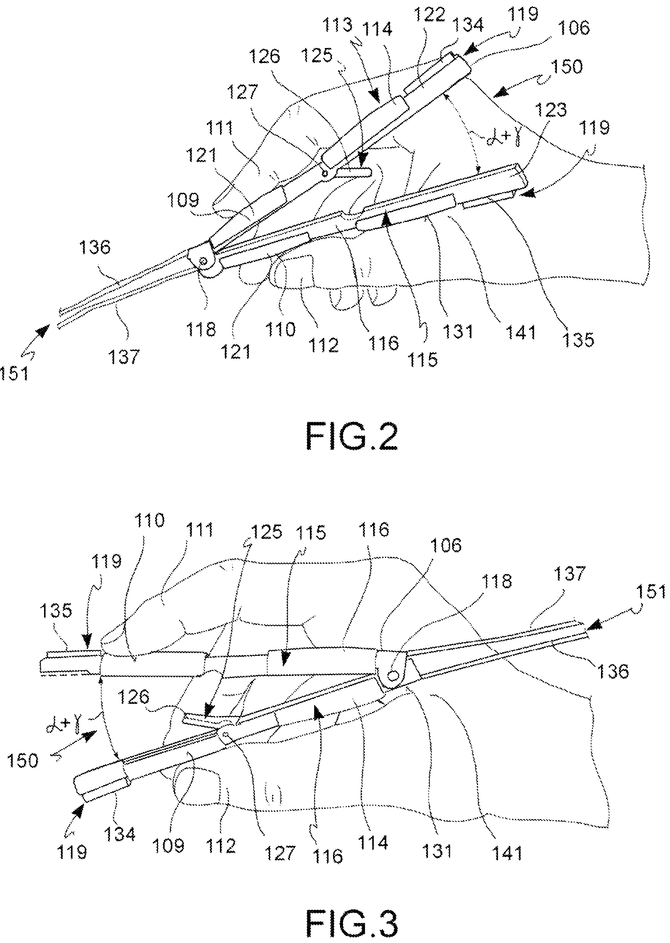

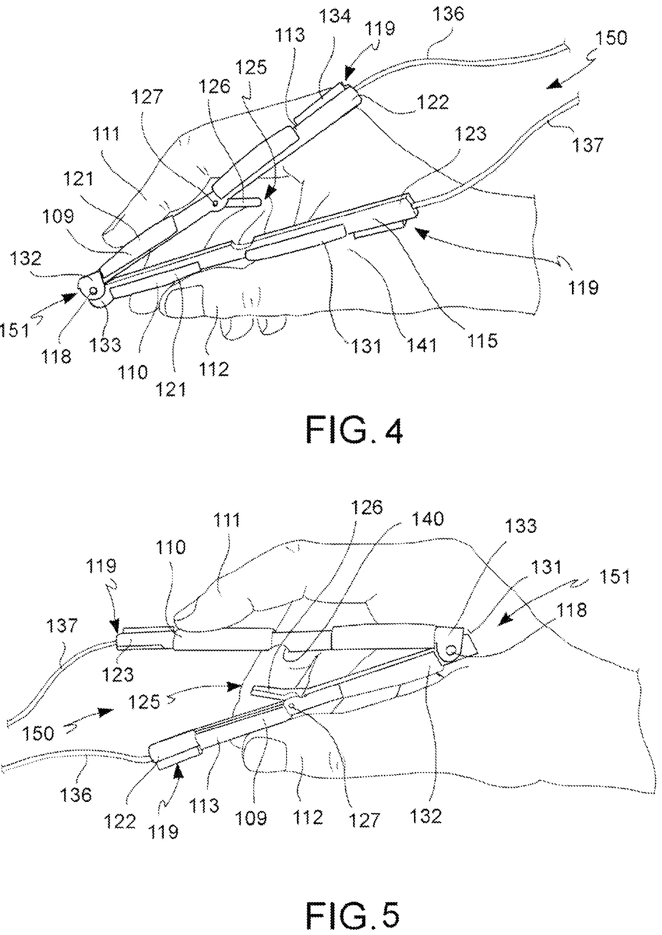

- figures 2 to 5 are perspective views showing master controller assembly, according to some embodiments, hand-held by surgeon;

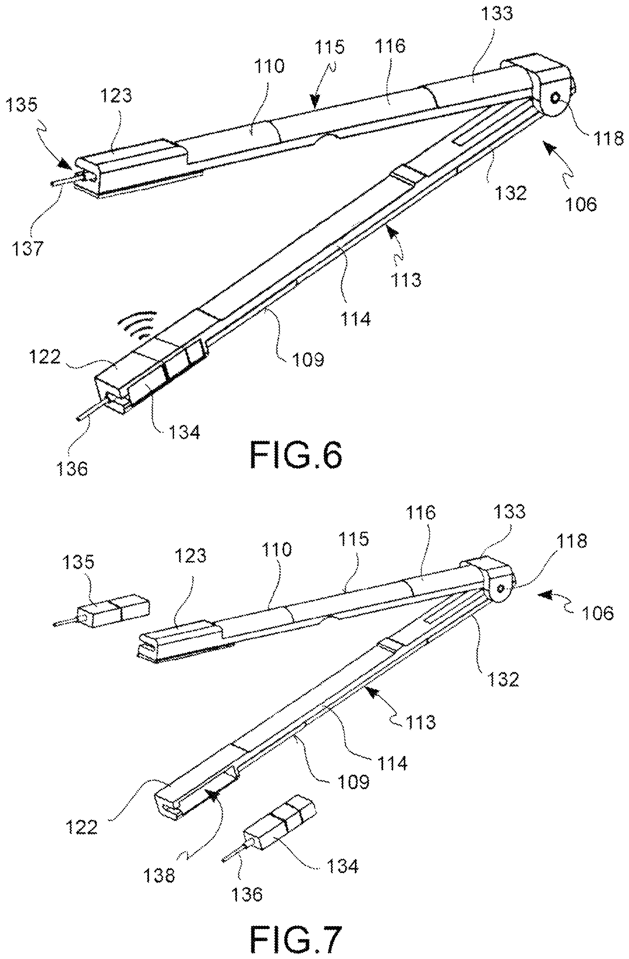

- figure 6 is a perspective view showing master controller assembly, according to an embodiment;

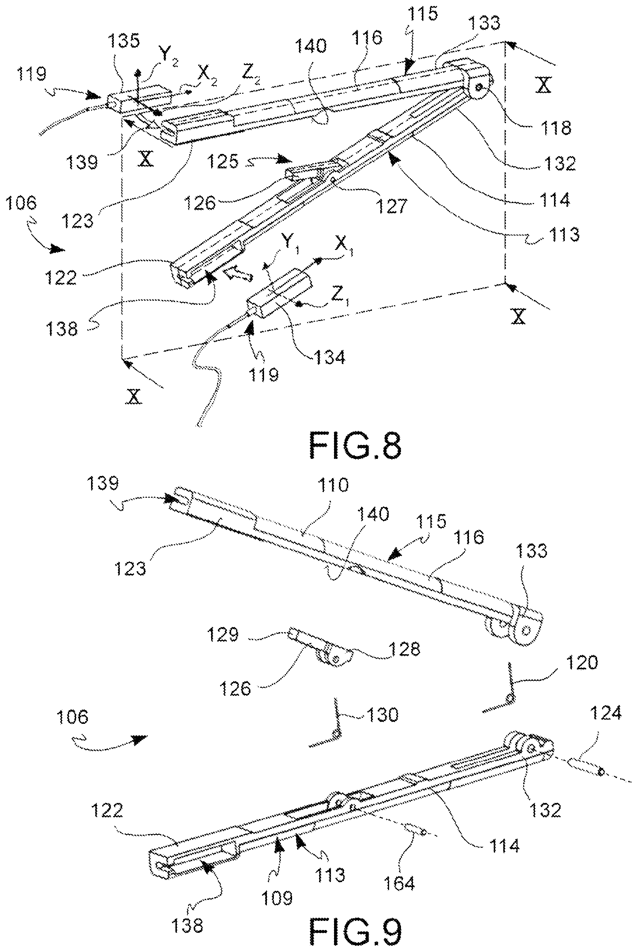

- figures 7 and 8 are perspective views of master controller assembly, according to some embodiments, showing as separated pieces a sensing assembly and a master input tool;
- figure 9 is a perspective exploded view of a master input tool, according to an embodiment;

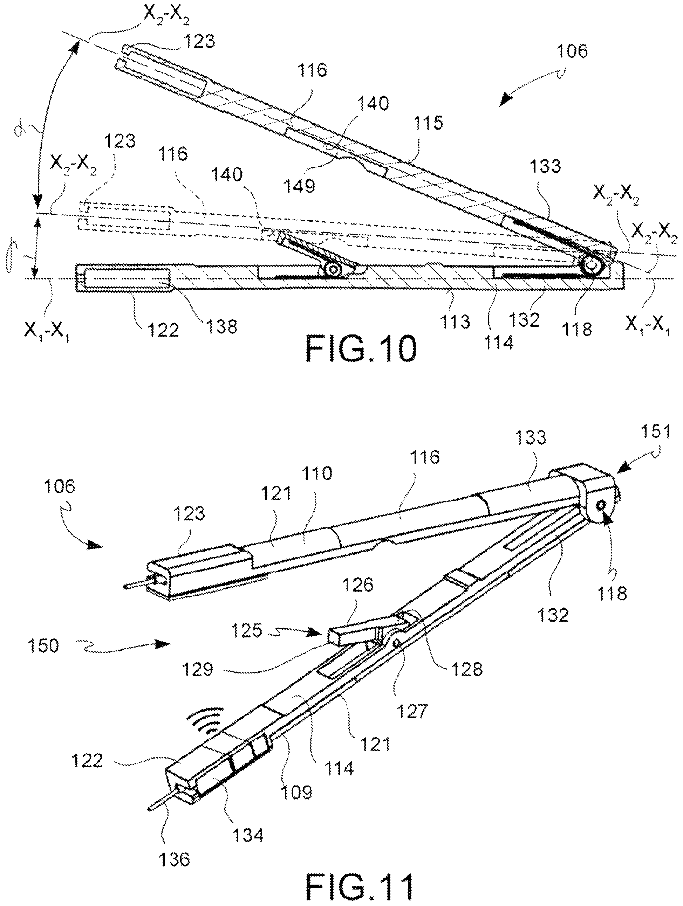

- figure 10 is a longitudinal cross-section of master input tool realized along the cut plane indicated with X-X-X-X in figure 8;
- figure 11 is a perspective view of master controller assembly, according to an embodiment;

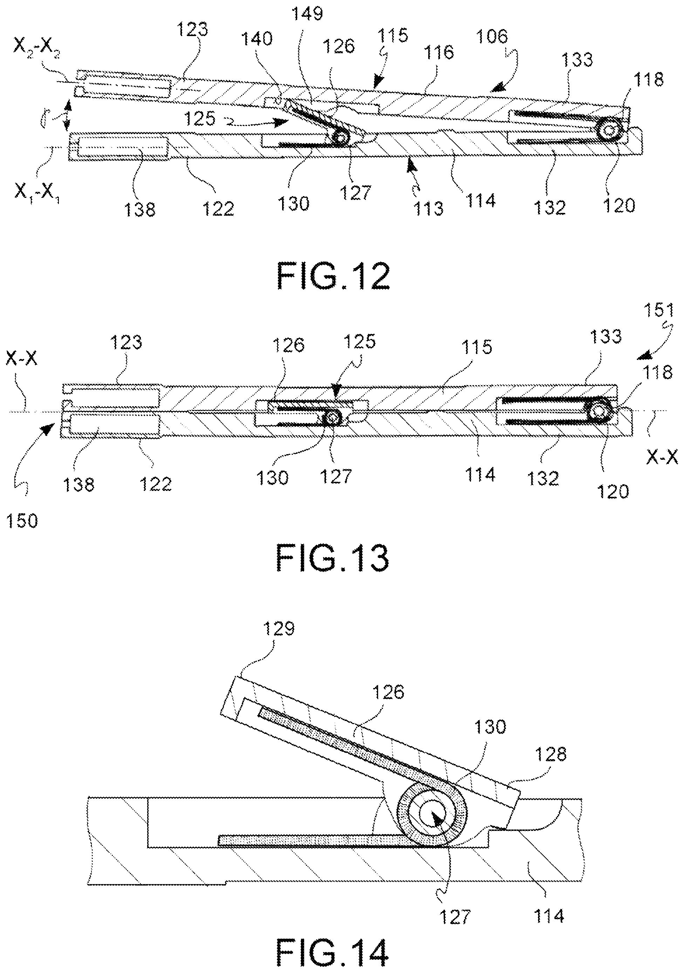

- figures 12 and 13 are longitudinal cross-sections of master input tool in closed positons, according to an embodiment;
- figure 14 is a longitudinal cross-section of trigger of master input tool, according to an embodiment;

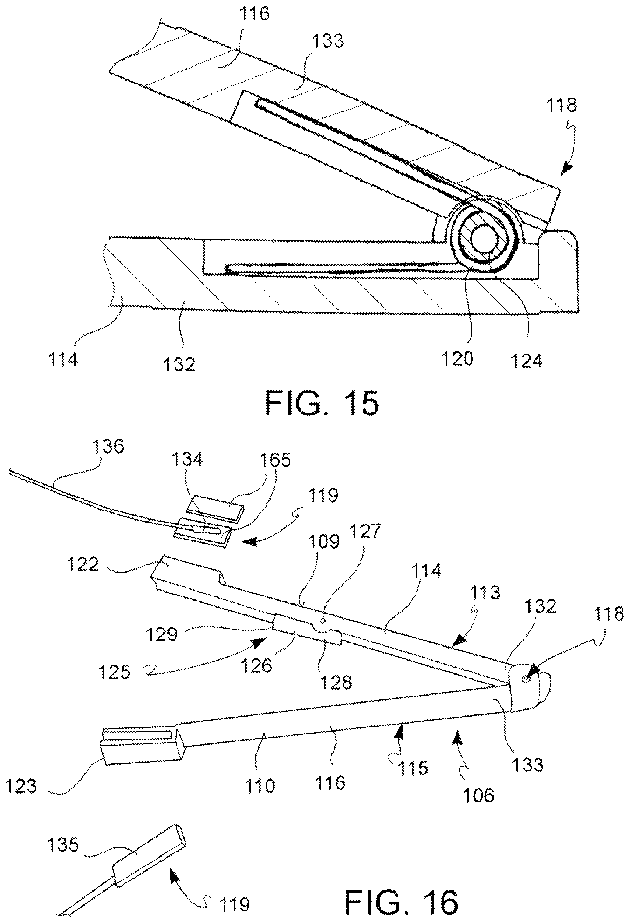

- figure 15 is a longitudinal cross-section of tool joint of master input tool, according to an embodiment;
- figure 16 is a perspective view of master controller assembly, according to an embodiment, wherein sensing assembly is shown as separate parts in respect of the master input tool;

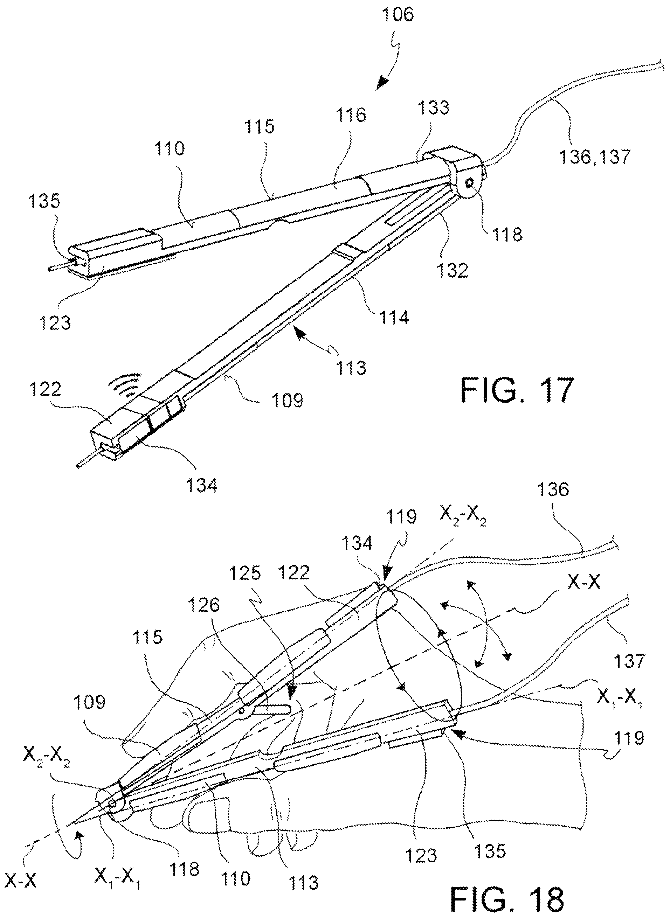

- figure 17 is a perspective view of master controller assembly, according to an embodiment;
- figure 18 is a perspective view of master controller assembly, according to an embodiment, being hand-held by surgeon;

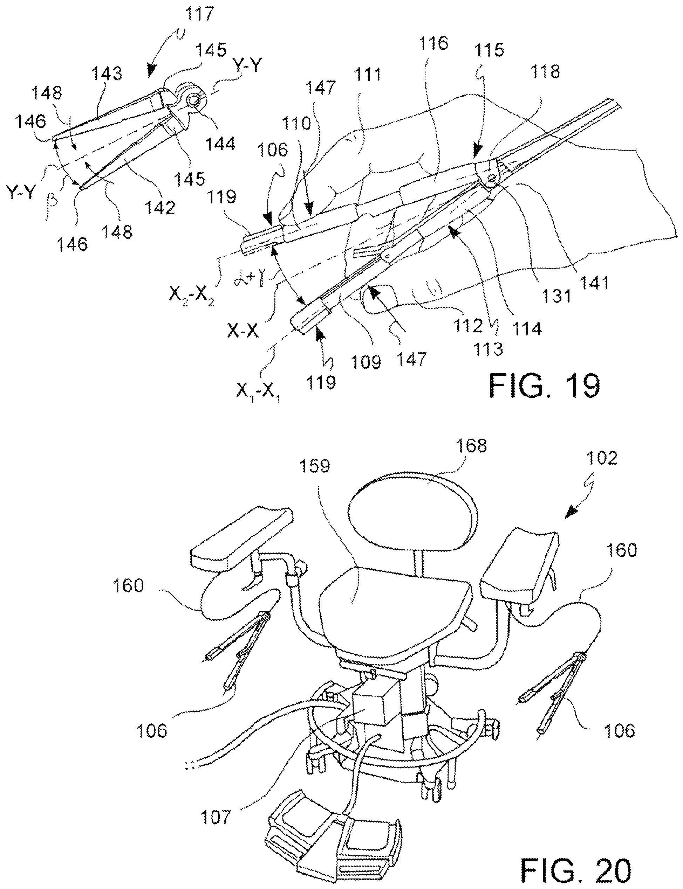

- figure 19 is a sketch in perspective view showing robotic surgery system comprising paired master controller assembly and slave surgical grip device, according to an embodiment;
- figure 20 is a perspective view showing master controller assembly, according to an embodiment;

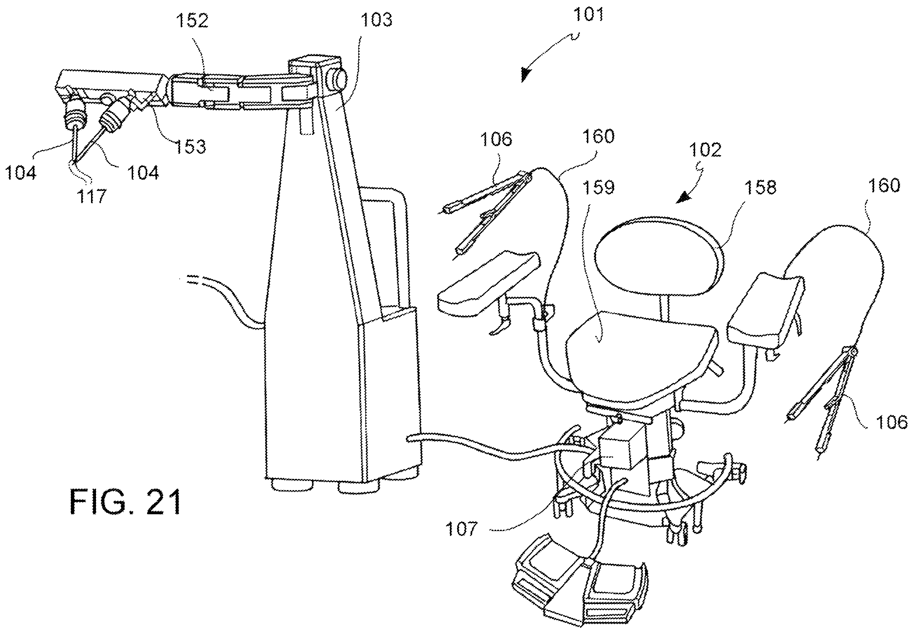

- figure 21 is a perspective view of robotic surgery system, according to an embodiment;

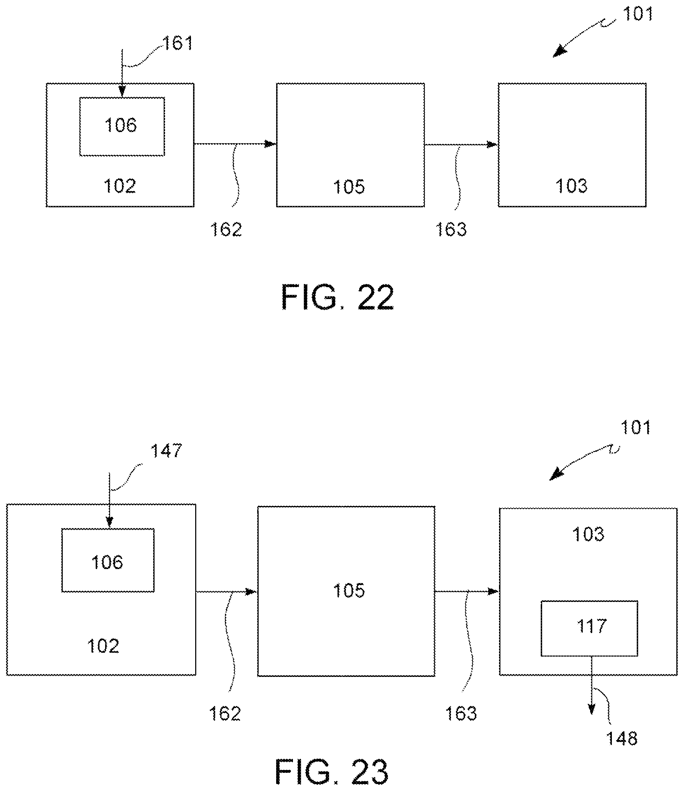

- figures 22 and 23 are block diagrams showing a robotic surgery assembly, according to some embodiments.

### . Detailed description of some embodiments

**.** According to a general embodiment, a robotic surgery system 101 comprises at least one master controller assembly 102, suitable to detect a manual command 161, and at least one slave robot assembly 103, comprising a slave surgical instrument 104 designed to operate on a patient anatomy.

**.** Said slave surgical instrument 104 comprises at least one surgical grip device 117 providing the slave surgical instrument 104 with a grip degree-of-freedom of motion.

**.** According to an embodiment, said surgical grip device 117 comprises a first elongated element of surgical grip device 142 and a second elongated element of surgical grip device 143, said first elongated element of surgical grip device 142 and said second elongated element of surgical grip device 143 being articulated in respect to one another forming a surgical grip joint 144, preferably said surgical grip joint 144 being a pin joint. Preferably, each of said first elongated element of surgical grip device 142 and said second elongated element of surgical grip device 143 comprises a joint portion of surgical grip device 145, forming at least a portion of said surgical grip joint 144, and a cantilevered free end of surgical grip device 146.

**.** According to a preferred embodiment, said robotic surgery system 101 comprises a control unit 105, suitable for receiving a first command signal 162 containing information about said manual command 161 and to transmit a second command signal 163 containing information about said manual command 161 to the slave robot assembly 103 in order to actuate said slave surgical instrument 104.

**.** According to a preferred embodiment, said master controller assembly 102 is paired along a master-slave pair to said slave surgical instrument 104. According to a preferred embodiment, said master controller assembly 102 and said slave surgical instrument 104 form, trough said control unit 105, a master-slave pair.

**.** Said master controller assembly 102 comprises at least one portable hand-held master input tool body 106 (or master input tool 106), suitable to be hand-held and manipulated by a surgeon from various locations of an operating arena during surgery. In this way, said master controller assembly 102 is provided with portability, for example during surgery within said operating arena. Preferably, said portable hand-held master input tool 106 is hand-held and manipulated by a surgeon from various locations of an operating arena during surgery. Preferably, said master input tool 106 receive said manual command.

**.** According to a preferred embodiment, the term "portable" referred to said master input tool indicates that the master input tool is capable to be carried or moved about, for example by the surgeon during surgery.

**.** According to a preferred embodiment, the term "hand-held" referred to said master input tool indicates that the master input tool is designed to be operated while held in a hand, for example the surgeon's hand.

**.** According to a preferred embodiment, the term "operating arena" refers to a portion of space at least partially surrounding a patient anatomy. Preferably, within the operating arena are comprised various locations beside the patient anatomy.

**.** According to a preferred embodiment, the term "manipulated" referred to said master input tool indicates that the master input tool can be treated or operated with or as if with hands.

**.** According to a preferred embodiment, said master input tool 106 is paired along a master-slave pair to said slave surgical instrument 104. According to a preferred embodiment, said master input tool 106 and said slave surgical instrument 104 form, trough said control unit 105, a master-slave pair.

**.** Said master controller assembly 102 is operatively connected to said slave robot assembly 103. According to an embodiment, said master controller assembly 102 is connected to said slave robotic assembly 103 by means of electromagnetic communication.

**.** Said master input tool 106 comprise at least one manipulandum surface 109, 110, designed to be hand-held by the surgeon's fingers 111, 112. In this way, the portability of the master input tool 106 is enhanced.

**.** Said master input tool 106 is mechanically unconstrained from said slave robot assembly 103, in such way that said master input tool 106 is, preferably naturally, movable, rotatable and spinnable by a surgeon.

**.** Said master input tool 106 is mechanically ungrounded.

**.** According to an embodiment, said master input tool is unsuitable for providing force feedback.

**.** Said at least one manipulandum surface 109, 110 is a convex surface, so that said master input tool 106 can be rolled between surgeon's fingers 111, 112 around a tool longitudinal axis X-X.

**.** According to a preferred embodiment, said master input tool 106 comprises a first elongated element 113 having a first element elongated body 114, wherein said first element elongated body 114 is a rigid body. According to a preferred embodiment, the terminology "rigid body" means that such a body is deficient or devoid of flexibility. According to an embodiment, the terminology "rigid body" means that such a body is unable to provide elastically flexural deformation when in operative conditions.

**.** According to an embodiment, said first element elongated body 114 defines a first element direction X1-X1, substantially coincident with the axis of longitudinal development of said first element elongated body 114.

**.** According to a preferred embodiment, said master input tool 106 comprises a second elongated element 115 having a second element elongated body 116, wherein said second element elongated body 116 is a rigid body.

**.** According to an embodiment, said second element elongated body 116 defines a second element direction X2-X2, substantially coincident with the axis of longitudinal development of said second element elongated body 116.

**.** According to a preferred embodiment, said master input tool 106 comprises a tool joint 118 connecting and articulating said first element elongated body 114 and said second element elongated body 116, providing a single degree of freedom of motion between said first element elongated body 114 and said second element elongated body 116.

**.** According to an embodiment, said single degree of freedom of motion between said first element elongated body 114 and said second element elongated body 116 lies in a predefined plane.

**.** According to a preferred embodiment, said master controller assembly 102 comprises at least one sensing assembly 119 detecting at least the mutual position, preferably the mutual position and the relative orientation, of said first element elongated body 114 and said second element elongated body 116. In this way, a gripping pressure action 147 exerted by the surgeon's fingers 111, 112 on said master input tool 106 moving said first element elongated body 114 and said second element elongated body 116 close one another other, determines a paired slave grip motion 148 of said surgical grip device 117.

**.** Thanks to the fact that said first element elongated body 114 and said second element elongated body 116 are both rigid bodies, the sensing resolution of said sensing assembly 119 is improved in respect of known solutions.

**.** According to an embodiment, said manual command 161 comprises said gripping pressure action 147.

**.** According to an embodiment, said paired slave grip motion 148 moves said first elongated element of surgical grip device 142 and a second elongated element of surgical grip device 143 close to one another.

**.** According to an embodiment, said gripping pressure action 147 is exerted by the surgeon's fingers 111, 112 on said at least one manipulandum surface 109, 110 of the master input tool 106.

**.** According to an embodiment, said sensing assembly 119 comprises at least one capacitive incremental position sensor, for example a capacitive encoder.

**.** According to an embodiment, said robotic surgery system 201, preferably said master controller assembly 102, comprises at least one field generator 107 generating a predefined field volume . According to a preferred embodiment, said at least one filed generator 107 generates a magnetic field.

**.** According to an embodiment, said at least one sensing assembly 119 detects at least the position, preferably at least the position and the orientation, of said master input tool 106 within said predefined field volume .

**.** According to an embodiment, said field generator 107 defines a reference zero point X0,Y0,Z0 integral with said field generator 107, and wherein said at least one sensing assembly 119 detecting the generated field local vector X1,Y1,Z1;X2,Y2,Z2, determines at least the position of said sensing assembly 119. In this way, sensing assembly 119 determines at least the position of said master tool assembly 106 integral with said sensing assembly 119within said predefined field volume.

**.** According to an embodiment, said master controller assembly 102 is operatively connected to said slave robot assembly 103 by means of a wired electric connection.

**.** According to an embodiment, said master controller assembly 102 is operatively connected to said slave robot assembly 103 by means of a wireless connection.

**.** According to an embodiment, said master input tool 106 is mechanically unconstrained from both the field generator 107 and the slave robot assembly 103, in such way that said master input tool 106 being naturally movable, rotatable and spinnable by a surgeon within said predefined filed volume .

**.** According to an embodiment, at least one of said first element elongated body 114 and said second element elongated body 116 comprises said at least one manipulandum surface 109, 110.

**.** According to a preferred embodiment, each of said first element elongated body 114 and said second element elongated body 116 comprises said at least one manipulandum surface 109, 110. In this way, said first element elongated body 114 comprises a first manipulandum surface 109, and said second element elongated body 116 comprises a second manipulandum surface 110.

**.** According to an embodiment, said at least one manipulandum surface 109, 110 comprises a friction enhanced portion 121, suitable for improving the grip of surgeon's fingers 111, 112 thereon.

**.** According to an embodiment, said at least one manipulandum surface 109, 110 is a portion of a cylindrical surface. In this way, the rollability of the master input tool 106 around a tool longitudinal axis X-X is enhanced.

**.** According to an embodiment, said first manipulandum surface 109 and said second manipulandum surface 110 cooperate to form at least a portion of a cylindrical surface. In this way, the rollability of the master input tool 106 around a tool longitudinal axis X-X is enhanced.

**.** According to an embodiment, said tool joint 118 is a hinge providing a single degree of freedom of motion of rotation between said first element elongated body 114 and said second element elongated body 116. In this way, said first element elongated body 114 and said second element elongated body 116 are movable in respect of one another of an angular movement.

**.** According to an embodiment, a master gripping angle α+γ is defined as the angle between said first element elongated body 114 and said second element elongated body 116. According to an embodiment, the angle between said first element elongated body 114 and said second element elongated body 116 defines a master gripping angle α+γ.

**.** According to an embodiment, said first element elongated body 114 and said second element elongated body 116 are movable in respect of one another of angular motion between at least one open position, wherein said master gripping angle α+γ is greater than a predefined grip threshold angle γ, and at least one closed position, wherein said master gripping angle α+γ is smaller than a predefined grip threshold angle γ.

**.** According to a preferred embodiment, said master gripping angle α+γ is equal to or lower than 60 degrees, when said first element elongated body 114 and said second element elongated body 116 are in an open position. Preferably, said master gripping angle α+γ is equal to or lower than 45 degrees, when said first element elongated body 114 and said second element elongated body 116 are in an open position. said master gripping angle α+γ is equal to or lower than 35 degrees, when said first element elongated body 114 and said second element elongated body 116 are in an open position.

**.** According to an embodiment, said tool joint 118 is a pin joint providing a single degree of freedom of motion, preferably of angular motion, of rotation between said first element elongated body 114 and said second element elongated body 116.

**.** According to an embodiment, the tool longitudinal axis X-X is defined as being coincident with the bisector of said master gripping angle α+γ.

**.** According to an embodiment, the tool longitudinal axis X-X is defined as the set of points that are equidistant from said fist element direction X1-X1 and from said second element direction X2-X2.

**.** According to an embodiment, the tool longitudinal axis X-X is defined as the axis of longitudinal development of said master input tool 106, when said first element elongated body 114 and said second element elongated body 116 are in a closed position.

**.** According to an embodiment, said first element elongated body 114 comprises a first element joint portion 132, forming a portion of said tool joint 118, and a first element cantilevered portion 122, located opposite to said first element joint portion 132 along the first element direction X1-X1.

**.** According to an embodiment, said second element elongated body 116 comprises a second element joint portion 133, forming a portion of said tool joint 118, and a second element cantilevered portion 123, located opposite to said second element joint proximal portion 132 along the second element direction X2-X2.

**.** Preferably, said first element cantilevered portion 122 form a free end, and said second element cantilevered portion 123 form a free end.

**.** According to an embodiment, the relative spatial position of said first element cantilevered portion 122 and said second element cantilevered portion 123 is rigidly determined by said master gripping angle α+γ width. In this way, the sensing resolution of said sensing assembly 119 is enhanced.

**.** According to a preferred embodiment, said first element joint portion 132 and said second element joint portion 133 cooperate to form said tool joint 118.

**.** According to an embodiment, said first element joint portion 132 and said second element joint portion 133 are constrained one another by means of a tool joint pin 124 to form said tool joint 118.

**.** According to an embodiment, said first element cantilevered portion 122 and said second element cantilevered portion 123 are located at a predefined distance from said tool joint 118 along said first element elongated body 114 and said second element elongated body 116, respectively.

**.** According to an embodiment, said sensing assembly 119 comprises at least one joint sensor, preferably an encoder, located within said tool joint 118.

**.** According to an embodiment, said sensing assembly 119 comprises at least one proximity sensor 166 and at least one target object 167, said proximity sensor 166 cooperating with said target object 167 to detect at least the mutual position, preferably the mutual position and relative orientation, of said first elongated element and said second elongated element. Preferably, one between said first element elongated body and said second element elongated body comprises said proximity sensor and the other comprises said target object.

**.** According to an embodiment, said master input tool 106 comprises at least one joint spring 120 biasing at least said first element cantilevered portion 122 of said first element elongated body 114 away from said second element cantilevered portion 123 of said second element elongated body 116, along said single degree of freedom of motion.

**.** According to an embodiment, said joint spring 120 angularly biases said first element elongated body 114 and said second element elongated body 116 towards said at least one open position.

**.** According to an embodiment, said joint spring 120 is interposed between said first element elongated body 114 and said second element elongated body 116.

**.** According to an embodiment, said joint spring 120 is interposed between said first element joint portion 122 and said second element joint portion 123.

**.** According to an embodiment, said joint spring 120 is a torsional spring.

**.** According to an embodiment, said joint spring 120 is an axial spring.

**.** According to an embodiment, said joint spring 120 is located around said tool joint 118. According to an embodiment, said joint spring 120 is located around a tool joint pin 124 of said tool joint 118.

**.** According to an embodiment, said joint spring 120 exerts an elastic bias action directed to increase the master gripping angle α+γ.

**.** According to an embodiment, each of said first element elongated body 114 and said second element elongated body 116 is made in single piece.

**.** According to an embodiment, each of said first element elongated body 114 and said second element elongated body 116 is made of polymeric material.

**.** According to an embodiment, each of said first element elongated body 114 and said second element elongated body 116 is made by molding, preferably by injection molding. In this way is reduced the number of parts to be assembled together to form said master input tool 106.

**.** According to an embodiment, the angle between said first elongated element of surgical grip device 142 and said second elongated element of surgical grip device 143 is equal to the paired, along a master-slave pair, master gripping angle α+γ.

**.** According to an embodiment, said first elongated element of surgical grip device 142 and said second elongated element of surgical grip device 143 define a slave gripping angle β therebetween. Preferably, said first elongated element of surgical grip device 142 and said second elongated element of surgical grip device 143 of said slave surgical grip device 117 are movable in respect to one another between at least one open position, wherein said slave gripping angle β is greater than a predefined slave grip threshold, and at least one closed position, wherein said slave gripping angle β is lower than said predefined slave grip threshold, preferably substantially equal to zero. Preferably, when said first elongated element of surgical grip device 142 and said second elongated element of surgical grip device 143 are in a closed position, said first elongated element of surgical grip device 142 and said second elongated element of surgical grip device 143 are aligned, preferably along a slave grip device longitudinal axis Y-Y.

**.** According to an embodiment, when said first element elongated body 114 and said second element elongated body 116 are in a closed position, the paired slave surgical grip device 117 is in a closed position.

**.** According to a preferred embodiment, said master input tool 106 comprises a grip force detector device 125, detecting the gripping pressure action 147 exerted by the surgeon's fingers 111, 112 moving said first element elongated body 114 and said second element elongated body 116 close one another other below said predefined grip threshold angle γ.

**.** According to a preferred embodiment, said grip force detector device 125 of said master input tool 106 detects the gripping pressure action 147 when said master gripping angle is lower that said grip threshold angle γ.

**.** According to an embodiment, when the gripping pressure action 147 exerted by the surgeon's fingers 111, 112 moves said first element elongated body 114 and said second element elongated body 116 close one another other below said predefined grip threshold angle γ, determines a paired grip force increase exerted by said surgical grip device 117. In this way, the surgeon is allowed to be aware when the slave surgical grip device 117 is cutting at least a portion of a patient anatomy by mechanical force feedback.

**.** According to an embodiment, said grip force detector device 125 comprise at least one trigger 126 rotatably connected to said first element elongated body 114 forming a trigger joint 127.

**.** According to an embodiment, said trigger joint 127 is a pin joint comprising a trigger pin 164. According to an embodiment, said trigger joint 127 is an hinge.

**.** According to an embodiment, said trigger 126 comprises a trigger root 128 that forms a portion of said trigger joint 127 and a trigger free end 129 extending cantilevered in respect of said trigger joint 127.

**.** According to an embodiment, said grip force detector device 125 comprises at least one grip spring 130 biasing said trigger free end 129 away from said first element elongated body 114, so that said trigger 126 extends cantilevered facing said second element elongated body 116.

**.** According to an embodiment, when said trigger free end 129 is urged towards said first element elongated body 114 by means of the gripping pressure action exerted by the surgeon's fingers 111, 112, said grip spring 130 exerts an elastic return action directed to contrast said gripping pressure action exerted by the surgeon's fingers 111, 112, in such way to make the surgeon aware of said paired grip force increase exerted by said slave grip device 117 by means of mechanical force feedback.

**.** According to an embodiment, said second element elongated body 116 comprises a trigger abutment portion 140, that forms an abutment wall for the trigger free end 129, when said gripping pressure action exerted by the surgeon's fingers 111, 112 moves said first element elongated body 114 and said second element elongated body 116 close one another other below a predefined grip threshold γ.

**.** According to an embodiment, said trigger abutment portion 140 defines a trigger seat 149 for receiving at least a portion of said trigger 126 when said master input tool 106 is in a closed position.

**.** According to an embodiment, said trigger abutment portion 140 defines a trigger seat 149, suitable for receiving at least said trigger free end 129, when said gripping pressure action exerted by the surgeon's fingers 111, 112 moves said first element elongated body 114 and said second element elongated body 116 close one another other below a predefined grip threshold γ.

**.** According to an embodiment, said grip force detector device 125 comprises at least one load cell.

**.** According to an embodiment, said sensing assembly 119 comprises at least one first sensor 134. Preferably, said first sensor 134 is integral with said first elongated element 113, preferably integral with said first element elongated body 114.

**.** According to the invention, said first element elongated body 114 delimits at least one first slot 138 receiving at least a portion of said sensing assembly 119. According to the invention, said at least one first slot 138 receives at least a portion of said sensing assembly 119 in a detachable manner, so that the master input tool 106 comprising or devoid of said sensing assembly 119 is disposable.

**.** According to an embodiment, said sensing assembly 119 comprises at least one sterile sensor container 165, for example a plastic bag or a plastic box and/or the like, enclosing at least one of said first sensor 134 or said second sensor 135. In this way, sensor assembly 119 sterility is achievable avoiding to require sensor 134, 135 replacement after a single surgery. Thereby, master input tool body 106 may be made disposable and sensors 134, 135 can be utilized multiple times because their sterility is preserved. Preferably, also the wired connections 136, 137 to sensors 134, 135 are enclosed by sterile boxes 165 or appendix thereof.

**.** According to an embodiment, said first slot 138 receives said first sensor 134.

**.** According to an embodiment, said first sensor 134 is operatively connected to said field generator 107 by means of a first sensor connection 136. According to an embodiment, said first sensor connection 136 is a wired connection. According to an embodiment, said first sensor connection 136 is a wireless connection.

**.** According to a preferred embodiment, said sensing assembly 119 comprises at least one second sensor 135. Preferably, said second sensor 135 is integral with said second elongated element 115.

**.** According to an embodiment, said second element elongated body 116 delimits at least one second slot 139 receiving at least a portion of said sensing assembly 119. According to an embodiment, said second slot 139 receives at least a portion of said sensing assembly 119 in a detachable manner, so that the master input tool 106 comprising or devoid of said sensing assembly 119 is disposable.

**.** According to an embodiment, said second slot 139 receives said second sensor 135.

**.** According to an embodiment, said second sensor 135 is operatively connected to said field generator 107 by means of a second sensor connection 137. According to an embodiment, said second sensor connection 137 is a wired connection. According to an embodiment, said second sensor connection 137 a wireless connection.

**.** According to an embodiment, said first slot 138 faces opposite in respect of said second slot 139, so that a unique arrangement of said sensing assembly 119 is allowed. In this way, the chances of misplacing sensing assembly 119 are significantly reduced.

**.** According to an embodiment, said slots 138, 139 comprise at least one flag element, for example a notch and/or the like to signal whether the sensor 134, 135 is operatively received in the respective slot 138. 139.

**.** According to an embodiment, said slots 138, 139 have different flag element to each other so that a sensor 134, 135 can be operatively connected to only one of the slots 138, 139.

**.** According to an embodiment, the arrangement of said slots 138, 139 is asymmetric. According to an embodiment, the arrangement of said sensors 134, 135 is asymmetric.

**.** According to an embodiment, said first slot 138 is opposite to said second slot 139 with respect of said tool longitudinal axis X-X.

**.** According to an embodiment, said slots 138, 139 have substantially the same shape and size.

**.** According to an embodiment, said slots 138, 139 have substantially the shape of a parallelepiped.

**.** According to an embodiment, said slots 138, 139 are provided near the free end portion of each elongated body 114, 116, so that to have the maximum linear displacement keeping constant the angular displacement, and sensors 134, 135 are received in respective slots 138, 139.

**.** According to an embodiment, said slots 138, 139 are provided at maximum distance from the tool joint 118, so that to have the maximum linear displacement keeping constant the angular displacement and sensors 134, 135 are received in respective slots 138, 139.

**.** For example, the slots 138, 139 and sensors 134, 135 are provided near or at the distal end of each elongated body 114, 116, when the tool joint 118 is near or at the proximal end thereof.

**.** According to an embodiment, said first element cantilevered portion 122 and said second element cantilevered portion 123 of said master input tool 106 define a first longitudinal side 150, and wherein a second longitudinal side 151 is defined opposite to said first longitudinal side 150 in respect of said tool joint 118.

**.** According to an embodiment, said first sensor connection 136 and said second sensor connection 137 are both wired connections, and wherein the wires of said first sensor connection 136 and said second sensor connection 137 are both gathered on a same longitudinal side 150;151 of said master input tool 106. In this way the encumber of said sensor connections is reduced.

**.** According to an embodiment, said master input tool 106 comprises at least one back-of-hand resting portion 131, designed to touch at least a portion of the surgeon's back-of-hand 141, when in operative conditions.

**.** According to an embodiment, the position of said manipulandum surface 109, 110 along the tool longitudinal axis X-X is interposed between said first tool longitudinal side 150 and said sensing assembly 119. According to an embodiment, the position of said manipulandum surface 109, 110 along the tool longitudinal axis X-X is interposed between said first tool longitudinal side 150 and said force detector device 125.

**.** According to an embodiment, said first element elongated body 114 comprises at least one manipulandum surface 109,110, said manipulandum surface 109,110 is located along said first elongated element direction X1-X1 between said first element cantilevered portion 122 and said trigger joint 127. According to an embodiment, said first element elongated body 114 comprises at least one manipulandum surface 109, 110 said manipulandum surface 109,110 is located along said first elongated element direction X1-X1 between said first element joint portion 132 and said trigger joint 127.

**.** According to an embodiment, the position of said manipulandum surface 109, 110 along the tool longitudinal axis X-X is interposed between said second tool longitudinal side 151 and said sensing assembly 119. According to an embodiment, the position of said manipulandum surface 109, 110 along the tool longitudinal axis X-X is interposed between said second tool longitudinal side 151 and said force detector device 125.According to an embodiment, said slave robot assembly 103 further comprises at least one surgical arm 152 manipulating said slave surgical instrument 104. According to an embodiment, said salve robot assembly 103 comprises at least one micromanipulator 153 manipulating said slave surgical instrument 104. Preferably, said at least one micromanipulator 153 is directly connected in series to said surgical arm 152 forming a kinematic chain with said surgical arm 152, said micromanipulator 153 manipulating said slave surgical instrument 104. According to an embodiment, at least two micromanipulators 153 are directly connected in series to said surgical arm 152 forming an at least two-branched kinematic chain with said surgical arm 153.

**.** According to an embodiment, said robotic surgery system 101 comprises at least one robot cart 154 comprising at least one cart ground contact unit 155 and a cart handle 156, said cart handle 156 being suitable for moving at least a portion of the robotic surgery system 101, preferably said slave robot assembly 103, at least within the operating arena. Preferably, said robot cart 154 forms a mechanical and structural support, preferably a movable mechanical and structural support, for the slave robot assembly 103.

**.** According to an embodiment, said robot cart 154 is connected to a power supply cable 157.

**.** According to an embodiment, said robot cart 154 comprises said control unit 105. . Preferably said control unit 105 is located integral said robot cart 154.

**.** According to an embodiment, said robot cart 154 comprises said filed generator 107.

**.** According to an embodiment, said master controller assembly 102 further comprises at least one surgical chair 158 comprising at least one seating surface 159 for the surgeon to seat thereon during surgery.

**.** According to an embodiment, said surgical chair 158 being mechanically unconstrained from the slave robot assembly 103, so as to prevent the propagation by mechanical contact of vibrational motion from the surgical chair 158 to the slave robot assembly 103. In this way, is reduced the risk of unwanted commands transmittal to the slave surgical robot 103, and particularly to said slave surgical instrument 104.

**.** According to an embodiment, said surgical chair 158 comprises said field generator 107 so that said field volume is integral with at least a portion of said surgical chair 158.

**.** According to an embodiment, said master input tool 106 is operatively connected to said surgical chair 158 by means of a chair operative connection 160. According to an embodiment, said chair operative connection 160 is a wired connection. According to an embodiment, said chair operative connection 160 is a wireless connection.

**.** According to a general embodiment, it is provided a master controller assembly 102 for a robotic surgery system 101, said robotic surgery system 101 further comprising a slave robot assembly 103 comprising a slave surgical instrument 104 having a surgical grip device 117 providing the slave surgical instrument 104 with a grip degree-of-freedom of motion.

**.** Said master controller assembly 102 comprises a master input tool body 106 and a sensing assembly 119, according to any one of the embodiments described above.

**.** According to a preferred embodiment, said master input tool 106 being suitable to be hand-held and manipulated by a surgeon from various locations of an operating arena during surgery, said master input tool 106 being suitable for receiving a manual command.

**.** According to a preferred embodiment, said master input tool 106 comprising at least one manipulandum surface 109, 110, designed to be hand-held by the surgeon's fingers 111, 112.

**.** According to a preferred embodiment, said master input tool 106 is mechanically unconstrained from said slave robot assembly 103, in such way that said master input tool 106 being naturally movable, rotatable and spinnable by a surgeon.

**.** According to a preferred embodiment, said at least one manipulandum surface 109, 110 is a convex surface, so that said master input tool 106 can be rolled between surgeon's fingers 111, 112 around a tool longitudinal axis X-X.

**.** According to a preferred embodiment, said master input tool 106 comprises a first elongated element 113 having an first element elongated body 114, wherein said first element elongated body 114 is a rigid body, and wherein said master input tool 106 comprises a second elongated element 115 having an second element elongated body 116, wherein said second element elongated body 116 is a rigid body.

**.** According to a preferred embodiment, said master input tool 106 comprises a tool joint 118 connecting and articulating said first element elongated body 114 and said second element elongated body 116, providing a single degree of freedom of motion between said first element elongated body 114 and said second element elongated body 116.

**.** According to a preferred embodiment, said master controller assembly 102 comprises at least one sensing assembly 119 detecting at least the mutual position of said first element elongated body 114 and said second element elongated body 116, so that a gripping pressure action exerted by the surgeon's fingers 111, 112 on said master input tool 106 moving said first element elongated body 114 and said second element elongated body 116 close one another other determines a paired grip motion of said surgical grip device 117.

**.** By virtue of the features described above, provided either separately or in combination, where applicable, in particular embodiments, it is possible to satisfy the sometimes contrasting needs disclosed above, and to obtain the aforesaid advantages, and in particular:
- it is provided a hand-held ungrounded master controller assembly of simple manufacturing and at the same time capable of accurate and reliable sensing;
- it is provided a master controller assembly where no need of structural constrain to the robot is required, and the connection to the robot may be wired for data transmission purposes;
- the surgeon is provided with improved freedom of motion and at the same time with a familiar tool for performing robotic surgery;
- it is provided a master controller assembly particularly suitable for robotic microsurgery;
- the mechanical features of the sensing assembly and of the slots receiving the sensors allow a unique arrangement of the sensor thereby avoiding misplacements resulting in an improved safety in respect of known solutions and at the same time without requiring high costs for manufacture;
- the master input tool body may be made disposable and the sensing assembly may be not.

**.** Those skilled in art may make many changes and adaptations to the embodiments described above or may replace elements with others which are functionally equivalent in order to satisfy contingent needs without however departing from the scope of the appended claims.

### LIST OF REFERENCES

| | |
|---|---|
| 101 | Robotic surgery system |
| 102 | Master controller assembly |
| 103 | Slave robot assembly |
| 104 | Slave surgical instrument, or surgical instrument |
| 105 | Control unit |
| 106 | Master input tool body, or master input tool |
| 107 | Field generator |
| 109 | First manipulandum surface |
| 110 | Second manipulandum surface |
| 111 | Surgeon finger |
| 112 | Further surgeon finger |
| 113 | First elongated element |
| 114 | First element elongated body, or first elongated element body |
| 115 | Second elongated element |
| 116 | Second element elongated body, or second elongated element body |
| 117 | Surgical grip device, or slave surgical grip device |
| 118 | Tool joint |
| 119 | Sensing assembly |
| 120 | Joint spring |
| 121 | Friction enhanced portion |
| 122 | First element cantilevered portion |
| 123 | Second element cantilevered portion |
| 124 | Tool joint pin |
| 125 | Grip force detector device |
| 126 | Trigger |
| 127 | Trigger joint |
| 128 | Trigger root |
| 129 | Trigger free end |
| 130 | Trigger spring |
| 131 | Back-of-the-hand resting portion |
| 132 | First element joint portion |
| 133 | Second element joint portion |
| 134 | First sensor |
| 135 | Second sensor |
| 136 | First sensor connection |
| 137 | Second sensor connection |
| 138 | First slot |
| 139 | Second slot |
| 140 | Trigger abutment portion |
| 141 | Surgeon's back-of-hand |
| 142 | First elongated element of surgical grip device |
| 143 | Second elongated element of surgical grip device |
| 144 | Surgical grip joint |
| 145 | Joint portion of surgical grip device |
| 146 | Free end of surgical grip device |
| 147 | Gripping pressure action |
| 148 | Paired slave grip motion |
| 149 | Trigger seat |
| 150 | First longitudinal side |
| 151 | Second longitudinal side |
| 152 | Surgical arm, or slave surgical arm |
| 153 | Micromanipulator |
| 154 | Robot cart |
| 155 | Cart ground contact unit |
| 156 | Cart handle |
| 157 | Power supply cable |
| 158 | Surgical chair |
| 159 | Seating surface |
| 160 | Chair operative connection |
| 161 | Manual command |
| 162 | First command signal |
| 163 | Second command signal |
| 164 | Trigger pin |
| 165 | Sterile sensor container |
| 166 | Proximity sensor |
| 167 | Target object |
| X-X | Tool longitudinal axis |
| X1-X1 | First element direction |
| X2-X2 | Second element direction |
| Y-Y | Slave grip device longitudinal axis |
| α+γ | Master gripping angle |
| β | Slave gripping angle |
| γ | Grip threshold angle |

## Claims

1. Master controller assembly (102) for a robotic surgery system (101), said robotic surgery system (101) further comprising a slave robot assembly (103) comprising a slave surgical instrument (104) having a surgical grip device (117) providing the slave surgical instrument (104) with a grip degree-of-freedom of motion, comprising:
- at least one master input tool (106) suitable to be hand-held and manipulated by a surgeon from various locations of an operating arena during surgery, and
- at least one sensing assembly (119);
wherein:
- said master input tool (106) being suitable for receiving a manual command;
- said master input tool (106) comprising at least one manipulandum surface (109, 110), designed to be hand-held by the surgeon's fingers (111, 112);
- said master input tool (106) is mechanically unconstrained from said slave robot assembly (103), in such way that said master input tool (106) being naturally movable, rotatable and spinnable by a surgeon;
- said at least one manipulandum surface (109, 110) is a convex surface, so that said master input tool (106) can be rolled between surgeon's fingers (111, 112) around a tool longitudinal axis (X-X); and wherein:
- said master input tool (106) comprises a first elongated element (113) having an first element elongated body (114), wherein said first element elongated body (114) is a rigid body;
- said master input tool (106) comprises a second elongated element (115) having an second element elongated body (116), wherein said second element elongated body (116) is a rigid body;
- said master input tool (106) comprises a tool joint (118) connecting and articulating said first element elongated body (114) and said second element elongated body (116), providing a single degree of freedom of motion between said first element elongated body (114) and said second element elongated body (116);
- said at least one sensing assembly (119) detecting at least the mutual position of said first element elongated body (114) and said second element elongated body (116), so that a gripping pressure action exerted by the surgeon's fingers (111, 112) on said master input tool (106) moving said first element elongated body (114) and said second element elongated body (116) close one another other determines a paired grip motion of said surgical grip device (117),
**characterized in that**
said master input tool (106) is mechanically ungrounded,
said first element elongated body (114) delimits at least one first slot (138) receiving at least a portion of said sensing assembly (119) in a detachable manner so that the master input tool (106) comprising or devoid of said sensing assembly (119) is disposable.

2. Master controller assembly (102) according to claim **1,** wherein said sensing assembly (119) comprises at least one first sensor (134); and said first slot (138) receives said first sensor (134) so that said first sensor (134) is integral with said first elongated element (113).

3. Master controller assembly (102) according to the preceding claim, wherein said first slot (138) receives said first sensor (134) in a detachable manner, so that the master input tool (106) comprising or devoid of said sensing assembly (119) is disposable.

4. Master controller assembly (102) according to any of the preceding claims from **2** to **3,** wherein said first sensor (134) is operatively connected to a master tracking device (107) by means of a first sensor connection (136), and wherein said first sensor connection (136) is a wired connection or a wireless connection.

5. Master controller assembly (102) according to any of the preceding claims, wherein said second element elongated body (116) delimits at least one second slot (139) receiving at least a portion of said sensing assembly (119).

6. Master controller assembly (102) according to the preceding claim, wherein said sensing assembly (119) comprises at least one second sensor (135); wherein
- said second slot (139) receives said second sensor (135).

7. Master controller assembly (102) according to the preceding claim wherein
- the at least one second slot (139) receives said second sensor (135) in a detachable manner, so that the master input tool (106) comprising or devoid of said sensing assembly (119) is disposable; and/or wherein
- said second sensor (135) is integral with said second elongated element (115); and/or wherein
- said second sensor (135) is operatively connected to a master tracking device (107) by means of a second sensor connection (137), and wherein said second sensor connection (137) is a wired connection or a wireless connection.

8. Master controller assembly (102) according to any of the preceding claims from 5 to 7, wherein said first slot (138) faces opposite in respect of said second slot (139), so that a unique arrangement of said sensing device (119) is allowed.

9. Master controller assembly (102) according to claim **2** and claim **6,** wherein said first slot (138) and said second slot (139) are provided near the free end portion of each elongated body (114, 116), so that to have the maximum linear displacement keeping constant the angular displacement, ; preferably wherein
- said first slot (138) and said second slot (139) are provided at maximum distance from the tool joint (118), so that to have the maximum linear displacement keeping constant the angular displacement ; preferably wherein
- said first slot (138) and said second slot (139) and said first sensor (134) and said second sensor (135)are provided near or at the distal end of each elongated body (114, 116), when the tool joint (118) is near or at the proximal end thereof.

10. Master controller assembly (102) according to claim **2** and claim **6,** wherein said sensing assembly (119) comprises at least one sterile sensor container (165), enclosing at least one of said first sensor (134) or said second sensor (135), so that to preserve sterility of the sensor assembly (119).

11. Master controller assembly (102) according to claim **2** and claim **6,** wherein said first sensor (134) is operatively connected to a master tracking device (107) by means of a first sensor connection (136), wherein said second sensor (135) is operatively connected to said master tracking device (107) by means of a second sensor connection (137), wherein said first sensor connection (136) and said second sensor connection (137) are both wired connections, and wherein the wires of said first sensor connection (136) and said second sensor connection (137) are both gathered on a same longitudinal side of said master input tool (106).

12. Master controller assembly (102) according to any of the preceding claims, wherein:
- each of said first element elongated body (114) and said second element elongated body (116) is made in single piece; preferably wherein
each of said first element elongated body (114) and said second element elongated body (116) is made of polymeric material; preferably wherein
each of said first element elongated body (114) and said second element elongated body (116) is made by molding.

13. Master controller assembly (102) according to any of the preceding claims, wherein:
- at least one of said first element elongated body (114) and said second element elongated body (116) comprises said at least one manipulandum surface (109, 110); preferably wherein
- each of said first element elongated body (114) and said second element elongated body (116) comprises said at least one manipulandum surface (109, 110); preferably wherein
- said at least one manipulandum surface (109, 110) comprises a friction enhanced portion (121), suitable for improving the grip of surgeon's fingers (111, 112) thereon; preferably wherein
said at least one manipulandum surface (109, 110) is a portion of a cylindrical surface.

14. Master controller assembly (102) according to any of the preceding claims, wherein:
said master input tool (106) comprises a grip force detector device (125), suitable for detecting the gripping pressure action exerted by the surgeon's fingers (111, 112) moving said first element elongated body (114) and said second element elongated body (116) close one another other below a predefined grip threshold angle (γ); preferably wherein
- when the gripping pressure action exerted by the surgeon's fingers (111, 112) moves said first element elongated body (114) and said second element elongated body (116) close one another other below said predefined grip threshold angle (γ) determines a paired grip force increase exerted by said surgical grip device (117); preferably wherein
said grip force detector device (125) comprises at least one grip spring (130) biasing a trigger free end (129) of a trigger (126) away from said first element elongated body (114), so that said trigger (126) extends cantilevered facing said second element elongated body (116).

15. Master controller assembly (102) according to any of the preceding claims, wherein
- said master controller assembly (102) comprises at least one field generator (107) generating a predefined field volume; and wherein
- said sensing assembly (119) detecting at least the position of said master input tool (106) within said predefined field volume.

16. Master controller assembly (102) according to any of the preceding claims,
wherein said sensing assembly (119) comprises at least one joint sensor, preferably an encoder, located within said tool joint (118).

17. Master controller assembly (102) according to any of the preceding claims, wherein said sensing assembly (119) detects at least the mutual position, and preferably the mutual position and the relative orientation, of said first element elongated body (114) and said second element elongated body (116).

18. Robotic surgery system (101) comprising:
- at least one master controller assembly (102) according to any one of the preceding claims;
- at least one slave robot assembly (103), comprising a slave surgical instrument (104) designed to operate on a patient anatomy, said slave surgical instrument (104) comprises at least one surgical grip device (117) providing the slave surgical instrument (104) with a grip degree-of-freedom of motion;
- a control unit (105), suitable for receiving a first command signal containing information about said manual command and to transmit a second command signal containing information about said manual command to the slave robot assembly (103) in order to actuate said slave surgical instrument (104).

19. Robotic surgery system (101) according to claim **18,** wherein said field generator (107) is a magnetic field generator.

## Patentansprüche

1. Master-Steuerungsanordnung (102) für ein robotisches Chirurgiesystem (101), wobei das robotische Chirurgiesystem (101) ferner eine Slave-Roboteranordnung (103) umfasst, die ein Slave-Chirurgieinstrument (104) mit einer chirurgischen Greifvorrichtung (117) umfasst, welche dem Slave-Chirurgieinstrument (104) einen Bewegungsfreiheitsgrad beim Greifen bereitstellt, umfassend:
- mindestens ein Master-Eingabewerkzeug (106), das dazu geeignet ist, von einem Chirurgen während der Operation in der Hand gehalten und von verschiedenen Standorten eines Operationssaals aus bedient zu werden, und
- mindestens eine Sensoranordnung (119);
wobei:
- das Master-Eingabewerkzeug (106) dazu geeignet ist, einen manuellen Befehl zu empfangen;
- das Master-Eingabewerkzeug (106) mindestens eine Manipulandum-Oberfläche (109, 110) umfasst, die dazu ausgelegt ist, von den Fingern (111, 112) des Chirurgen gehalten zu werden;
- das Master-Eingabewerkzeug (106) von der Slave-Roboteranordnung (103) mechanisch derart uneingeschränkt ist, dass das Master-Eingabewerkzeug (106) von einem Chirurgen auf natürliche Weise bewegt, geschwenkt und gedreht werden kann;
- die mindestens eine Manipulandum-Oberfläche (109, 110) eine konvexe Oberfläche ist, sodass das Master-Eingabewerkzeug (106) zwischen den Fingern (111, 112) des Chirurgen um eine Werkzeuglängsachse (X-X) gedreht werden kann; und wobei:
- das Master-Eingabewerkzeug (106) ein erstes längliches Element (113) mit einem ersten länglichen Elementkörper (114) umfasst, wobei der erste längliche Elementkörper (114) ein starrer Körper ist;
- das Master-Eingabewerkzeug (106) ein zweites längliches Element (115) mit einem zweiten länglichen Elementkörper (116) umfasst, wobei der zweite längliche Elementkörper (116) ein starrer Körper ist;
- das Master-Eingabewerkzeug (106) ein Werkzeuggelenk (118) umfasst, das den ersten länglichen Elementkörper (114) und den zweiten länglichen Elementkörper (116) verbindet und anlenkt, wodurch ein einziger Bewegungsfreiheitsgrad zwischen dem ersten länglichen Elementkörper (114) und dem zweiten länglichen Elementkörper (116) bereitgestellt wird;
- die mindestens eine Sensoranordnung (119) zumindest die gegenseitige Position des ersten länglichen Elementkörpers (114) und des zweiten länglichen Elementkörpers (116) erfasst, sodass eine von den Fingern (111, 112) des Chirurgen auf das Master-Eingabewerkzeug (106) ausgeübte Greifdruckwirkung, die den ersten länglichen Elementkörper (114) und den zweiten länglichen Elementkörper (116) einander annähert, eine gepaarte Greifbewegung der chirurgischen Greifvorrichtung (117) bestimmt,
**dadurch gekennzeichnet, dass**
das Master-Eingabewerkzeug (106) mechanisch nicht geerdet ist,
der erste längliche Elementkörper (114) mindestens einen ersten Schlitz (138) begrenzt, der mindestens einen Abschnitt der Sensoranordnung (119) abnehmbar aufnimmt, sodass das Master-Eingabewerkzeug (106), das die Sensoranordnung (119) umfasst oder nicht, entsorgt werden kann.

2. Master-Steuerungsanordnung (102) nach Anspruch 1, wobei die Sensoranordnung (119) mindestens einen ersten Sensor (134) umfasst; und der erste Schlitz (138) den ersten Sensor (134) aufnimmt, sodass der erste Sensor (134) einstückig mit dem ersten länglichen Element (113) gebildet ist.

3. Master-Steuerungsanordnung (102) nach dem vorhergehenden Anspruch, wobei der erste Schlitz (138) den ersten Sensor (134) abnehmbar aufnimmt, sodass das Master-Eingabewerkzeug (106), das die Sensoranordnung (119) umfasst oder nicht, entsorgt werden kann.

4. Master-Steuerungsanordnung (102) nach einem der vorhergehenden Ansprüche 2 bis 3, wobei der erste Sensor (134) mittels einer ersten Sensorverbindung (136) funktionsfähig mit einer Master-Verfolgungsvorrichtung (107) verbunden ist und wobei die erste Sensorverbindung (136) eine drahtgebundene Verbindung oder eine drahtlose Verbindung ist.

5. Master-Steuerungsanordnung (102) nach einem der vorhergehenden Ansprüche, wobei der zweite längliche Elementkörper (116) mindestens einen zweiten Schlitz (139) begrenzt, der mindestens einen Abschnitt der Sensoranordnung (119) aufnimmt.

6. Master-Steuerungsanordnung (102) nach dem vorhergehenden Anspruch, wobei die Sensoranordnung (119) mindestens einen zweiten Sensor (135) umfasst; wobei
- der zweite Schlitz (139) den zweiten Sensor (135) aufnimmt.

7. Master-Steuerungsanordnung (102) nach dem vorhergehenden Anspruch, wobei
- der mindestens eine zweite Schlitz (139) den zweiten Sensor (135) abnehmbar aufnimmt, sodass das Master-Eingabewerkzeug (106), das die Sensoranordnung (119) umfasst oder nicht, entsorgt werden kann; und/oder wobei
- der zweite Sensor (135) einstückig mit dem zweiten länglichen Element (115) gebildet ist; und/oder wobei
- der zweite Sensor (135) mittels einer zweiten Sensorverbindung (137) funktionsfähig mit einer Master-Verfolgungsvorrichtung (107) verbunden ist und wobei die zweite Sensorverbindung (137) eine drahtgebundene Verbindung oder eine drahtlose Verbindung ist.

8. Master-Steuerungsanordnung (102) nach einem der vorhergehenden Ansprüche 5 bis 7, wobei der erste Schlitz (138) dem zweiten Schlitz (139) gegenüberliegt, sodass eine eindeutige Anordnung der Sensorvorrichtung (119) ermöglicht wird.

9. Master-Steuerungsanordnung (102) nach Anspruch 2 und Anspruch 6, wobei der erste Schlitz (138) und der zweite Schlitz (139) nahe dem freien Endabschnitt jedes länglichen Körpers (114, 116) vorgesehen sind, sodass die maximale lineare Verschiebung die Winkelverschiebung konstant hält; vorzugsweise wobei
- der erste Schlitz (138) und der zweite Schlitz (139) in maximalem Abstand von dem Werkzeuggelenk (118) vorgesehen sind, sodass die maximale lineare Verschiebung die Winkelverschiebung konstant hält; vorzugsweise wobei
- der erste Schlitz (138) und der zweite Schlitz (139) und der erste Sensor (134) und der zweite Sensor (135) nahe dem oder am distalen Ende jedes länglichen Körpers (114, 116) vorgesehen sind, wenn sich das Werkzeuggelenk (118) nahe dem oder am proximalen Ende davon befindet.

10. Master-Steuerungsanordnung (102) nach Anspruch 2 und Anspruch 6, wobei die Sensoranordnung (119) mindestens einen sterilen Sensorbehälter (165) umfasst, der mindestens einen von dem ersten Sensor (134) oder dem zweiten Sensor (135) umschließt, um die Sterilität der Sensoranordnung (119) zu gewährleisten.

11. Master-Steuerungsanordnung (102) nach Anspruch 2 und Anspruch 6, wobei der erste Sensor (134) mittels einer ersten Sensorverbindung (136) funktionsfähig mit einer Master-Verfolgungsvorrichtung (107) verbunden ist, wobei der zweite Sensor (135) mittels einer zweiten Sensorverbindung (137) funktionsfähig mit der Master-Verfolgungsvorrichtung (107) verbunden ist, wobei die erste Sensorverbindung (136) und die zweite Sensorverbindung (137) beide drahtgebundene Verbindungen sind und wobei die Drähte der ersten Sensorverbindung (136) und der zweiten Sensorverbindung (137) beide auf derselben Längsseite des Master-Eingabewerkzeugs (106) zusammengefasst sind.

12. Master-Steuerungsanordnung (102) nach einem der vorhergehenden Ansprüche, wobei:
- der erste längliche Elementkörper (114) und der zweite längliche Elementkörper (116) jeweils aus einem einzelnen Stück bestehen; vorzugsweise wobei
der erste längliche Elementkörper (114) und der zweite längliche Elementkörper (116) jeweils aus einem Polymermaterial bestehen; vorzugsweise wobei
der erste längliche Elementkörper (114) und der zweite längliche Elementkörper (116) jeweils durch Formen hergestellt sind.

13. Master-Steuerungsanordnung (102) nach einem der vorhergehenden Ansprüche, wobei:
- mindestens einer des ersten länglichen Elementkörpers (114) und des zweiten länglichen Elementkörpers (116) die mindestens eine Manipulandum-Oberfläche (109, 110) umfasst; vorzugsweise wobei
- der erste längliche Elementkörper (114) und der zweite längliche Elementkörper (116) jeweils die mindestens eine Manipulandum-Oberfläche (109, 110) umfassen; vorzugsweise wobei
- die mindestens eine Manipulandum-Oberfläche (109, 110) einen reibungsverstärkten Abschnitt (121) umfasst, der dazu geeignet ist, den Halt der Finger (111, 112) des Chirurgen darauf zu verbessern; vorzugsweise wobei die mindestens eine Manipulandum-Oberfläche (109, 110) ein Abschnitt einer zylindrischen Oberfläche ist.

14. Master-Steuerungsanordnung (102) nach einem der vorhergehenden Ansprüche, wobei:
das Master-Eingabewerkzeug (106) eine Greifkraft-Erfassungsvorrichtung (125) umfasst, die dazu geeignet ist, die von den Fingern (111, 112) des Chirurgen ausgeübte Greifdruckwirkung, die den ersten länglichen Elementkörper (114) und den zweiten länglichen Elementkörper (116) einander annähert, unterhalb eines vorbestimmten Greifschwellenwinkels (y) zu erfassen; vorzugsweise wobei
- wenn die von den Fingern (111, 112) des Chirurgen ausgeübte Greifdruckwirkung den ersten länglichen Elementkörper (114) und den zweiten länglichen Elementkörper (116) unterhalb des vorbestimmten Greifschwellenwinkels (y) einander annähert, eine entsprechende Zunahme der von der chirurgischen Greifvorrichtung (117) ausgeübten gepaarten Greifkraft bestimmt wird; vorzugsweise wobei
die Greifkraft-Erfassungsvorrichtung (125) mindestens eine Grifffeder (130) umfasst, die ein freies Auslöserende (129) eines Auslösers (126) von dem ersten länglichen Elementkörper (114) weg vorspannt, sodass sich der Auslöser (126) auskragend in Richtung des zweiten länglichen Elementkörpers (116) erstreckt.

15. Master-Steuerungsanordnung (102) nach einem der vorhergehenden Ansprüche, wobei
- die Master-Steuerungsanordnung (102) mindestens einen Feldgenerator (107) umfasst, der ein vordefiniertes Feldvolumen erzeugt; und wobei
- die Sensoranordnung (119) zumindest die Position des Master-Eingabewerkzeugs (106) innerhalb des vordefinierten Feldvolumens erfasst.

16. Master-Steuerungsanordnung (102) nach einem der vorhergehenden Ansprüche,
wobei die Sensoranordnung (119) mindestens einen Gelenksensor, vorzugsweise einen Messgeber, umfasst, der innerhalb des Werkzeuggelenks (118) angeordnet ist.

17. Master-Steuerungsanordnung (102) nach einem der vorhergehenden Ansprüche, wobei die Sensoranordnung (119) zumindest die gegenseitige Position und vorzugsweise die gegenseitige Position und die relative Ausrichtung des ersten länglichen Elementkörpers (114) und des zweiten länglichen Elementkörpers (116) erfasst.

18. Robotisches Chirurgiesystem (101), umfassend:
- mindestens eine Master-Steuerungsanordnung (102) nach einem der vorhergehenden Ansprüche;
- mindestens eine Slave-Roboteranordnung (103), die ein Slave-Chirurgieinstrument (104) umfasst, das dazu ausgelegt ist, an der Anatomie eines Patienten zu operieren, wobei das Slave-Chirurgieinstrument (104) mindestens eine chirurgische Greifvorrichtung (117) umfasst, die dem Slave-Chirurgieinstrument (104) einen Bewegungsfreiheitsgrad beim Greifen bereitstellt;
- eine Steuereinheit (105), die dazu geeignet ist, ein erstes Befehlssignal zu empfangen, das Informationen über den manuellen Befehl enthält, und ein zweites Befehlssignal, das Informationen über den manuellen Befehl enthält, an die Slave-Roboteranordnung (103) zu übertragen, um das Slave-Chirurgieinstrument (104) zu betätigen.

19. Robotisches Chirurgiesystem (101) nach Anspruch 18, wobei der Feldgenerator (107) ein Magnetfeldgenerator ist.

## Revendications

1. Ensemble contrôleur principal (102) pour un système de chirurgie robotique (101), ledit système de chirurgie robotique (101) comprenant en outre un ensemble robot esclave (103) comprenant un instrument chirurgical esclave (104) ayant un dispositif de préhension chirurgical (117) fournissant à l'instrument chirurgical esclave (104) un degré de liberté de mouvement de préhension, comprenant :
- au moins un outil d'entrée principal (106) adapté pour être tenu à la main et manipulé par un chirurgien à partir de divers emplacements d'une salle d'opération pendant la chirurgie, et
- au moins un ensemble capteur (119) ;
dans lequel :
- ledit outil d'entrée principal (106) étant apte à recevoir une commande manuelle ;
- ledit outil d'entrée principal (106) comprenant au moins une surface de manipulateur (109, 110), conçue pour être tenue à la main par les doigts (111, 112) du chirurgien ;
- ledit outil d'entrée principal (106) n'est pas contraint mécaniquement par ledit ensemble robot esclave (103), de telle sorte que ledit outil d'entrée principal (106) soit naturellement mobile, rotatif et orientable par un chirurgie ;
- ladite au moins une surface de manipulateur (109, 110) est une surface convexe, de sorte que ledit outil d'entrée principal (106) peut être roulé entre les doigts (111, 112) du chirurgien autour d'un axe longitudinal de l'outil (X-X) ; et dans lequel :
- ledit outil d'entrée principal (106) comprend un premier élément allongé (113) ayant un premier corps allongé d'élément (114), dans lequel ledit premier corps allongé d'élément (114) est un corps rigide ;
- ledit outil d'entrée principal (106) comprend un deuxième élément allongé (115) ayant un deuxième corps allongé d'élément (116), dans lequel ledit deuxième corps allongé d'élément (116) est un corps rigide ;
- ledit outil d'entrée principal (106) comprend une articulation d'outil (118) reliant et articulant ledit premier corps allongé d'élément (114) et ledit deuxième corps allongé d'élément (116), offrant un seul degré de liberté de mouvement entre ledit premier corps allongé d'élément (114) et ledit deuxième corps allongé d'élément (116) ;
- ledit au moins un ensemble capteur (119) détectant au moins la position mutuelle dudit premier corps allongé d'élément (114) et dudit deuxième corps allongé d'élément (116), de sorte qu'une action de pression de préhension exercée par les doigts (111, 112) du chirurgien sur ledit outil d'entrée principal (106) déplaçant ledit premier corps allongé d'élément (114) et ledit deuxième corps allongé d'élément (116) l'un près de l'autre détermine un mouvement de préhension apparié dudit dispositif de préhension chirurgical (117),
**caractérisé en ce que**
ledit outil d'entrée principal (106) n'est pas mis à la terre mécaniquement,
ledit premier corps allongé d'élément (114) délimite au moins une première fente (138) recevant au moins une partie dudit ensemble capteur (119) de manière détachable de sorte que l'outil d'entrée principal (106) comprenant ou dépourvu dudit ensemble capteur (119) est jetable.

2. Ensemble contrôleur principal (102) selon la revendication 1, dans lequel ledit ensemble capteur (119) comprend au moins un premier capteur (134) ; et ladite première fente (138) reçoit ledit premier capteur (134) de sorte que ledit premier capteur (134) est solidaire dudit premier élément allongé (113).

3. Ensemble contrôleur principal (102) selon la revendication précédente, dans lequel ladite première fente (138) reçoit ledit premier capteur (134) de manière détachable, de sorte que l'outil d'entrée principal (106) comprenant ou dépourvu dudit ensemble capteur (119) est jetable.

4. Ensemble contrôleur principal (102) selon l'une quelconque des revendications précédentes 2 à 3, dans lequel ledit premier capteur (134) est fonctionnellement relié à un dispositif de suivi principal (107) au moyen d'une première liaison de capteur (136), et dans lequel ladite première liaison de capteur (136) est une liaison filaire ou une liaison sans fil.

5. Ensemble contrôleur principal (102) selon l'une quelconque des revendications précédentes, dans lequel ledit deuxième corps allongé d'élément (116) délimite au moins une deuxième fente (139) recevant au moins une partie dudit ensemble capteur (119).

6. Ensemble contrôleur principal (102) selon la revendication précédente, dans lequel ledit ensemble capteur (119) comprend au moins un deuxième capteur (135) ; dans lequel
- ladite deuxième fente (139) reçoit ledit deuxième capteur (135).

7. Ensemble contrôleur principal (102) selon la revendication précédente dans lequel
- l'au moins une deuxième fente (139) reçoit ledit deuxième capteur (135) de manière détachable, de sorte que l'outil d'entrée principal (106) comprenant ou dépourvu dudit ensemble capteur (119) est jetable ; et/ou dans lequel
- ledit deuxième capteur (135) est solidaire dudit deuxième élément allongé (115) ; et/ou dans lequel
- ledit deuxième capteur (135) est fonctionnellement relié à un dispositif de suivi principal (107) au moyen d'une deuxième liaison de capteur (137), et dans lequel ladite deuxième liaison de capteur (137) est une liaison filaire ou une liaison sans fil.

8. Ensemble contrôleur principal (102) selon l'une quelconque des revendications précédentes 5 à 7, dans lequel ladite première fente (138) est opposée par rapport à ladite deuxième fente (139), de sorte qu'une disposition unique dudit dispositif de détection (119) est autorisée.

9. Ensemble contrôleur principal (102) selon la revendication 2 et la revendication 6, dans lequel ladite première fente (138) et ladite deuxième fente (139) sont prévues près de la partie d'extrémité libre de chaque corps allongé (114, 116), de sorte que le déplacement linéaire maximal soit obtenu en maintenant constant le déplacement angulaire ; de préférence dans lequel
- ladite première fente (138) et ladite deuxième fente (139) sont prévues à une distance maximale de l'articulation d'outil (118), de sorte que le déplacement linéaire maximal soit obtenu en maintenant constant le déplacement angulaire ; de préférence, dans lequel
- ladite première fente (138) et ladite deuxième fente (139) et ledit premier capteur (134) et ledit deuxième capteur (135) sont prévus près de ou à l'extrémité distale de chaque corps allongé (114, 116), lorsque l'articulation d'outil (118) est près de ou à son extrémité proximale.

10. Ensemble contrôleur principal (102) selon la revendication 2 et la revendication 6, dans lequel ledit ensemble capteur (119) comprend au moins un récipient de capteur stérile (165), enfermant au moins l'un dudit premier capteur (134) ou dudit deuxième capteur (135), de manière à préserver la stérilité de l'ensemble capteur (119).

11. Ensemble contrôleur principal (102) selon la revendication 2 et la revendication 6, dans lequel ledit premier capteur (134) est fonctionnellement relié à un dispositif de suivi principal (107) au moyen d'une première liaison de capteur (136), dans lequel ledit deuxième capteur (135) est fonctionnellement relié audit dispositif de suivi principal (107) au moyen d'une deuxième liaison de capteur (137), dans lequel ladite première liaison de capteur (136) et ladite deuxième liaison de capteur (137) sont toutes deux des liaisons filaires, et dans lequel les fils de ladite première liaison de capteur (136) et de ladite deuxième liaison de capteur (137) sont tous deux rassemblés sur un même côté longitudinal dudit outil d'entrée principal (106).

12. Ensemble contrôleur principal (102) selon l'une quelconque des revendications précédentes, dans lequel :
- chacun dudit premier corps allongé d'élément (114) et dudit deuxième corps allongé d'élément (116) est réalisé d'une seule pièce ; de préférence dans lequel
chacun dudit premier corps allongé d'élément (114) et dudit deuxième corps allongé d'élément (116) est fait d'un matériau polymère ; de préférence dans lequel
chacun dudit premier corps allongé d'élément (114) et dudit deuxième corps allongé d'élément (116) est fabriqué par moulage.

13. Ensemble contrôleur principal (102) selon l'une quelconque des revendications précédentes, dans lequel :
- au moins l'un dudit premier corps allongé d'élément (114) et dudit deuxième corps allongé d'élément (116) comprend ladite au moins une surface de manipulateur (109, 110) ; de préférence dans lequel
- chacun dudit premier corps allongé d'élément (114) et dudit deuxième corps allongé d'élément (116) comprend ladite au moins une surface de manipulateur (109, 110) ; de préférence dans lequel
- ladite au moins une surface de manipulateur (109, 110) comprend une partie améliorée par frottement (121), adaptée pour améliorer la préhension des doigts (111, 112) du chirurgien sur celle-ci ; de préférence, ladite au moins une surface de manipulateur (109, 110) est une partie d'une surface cylindrique.

14. Ensemble contrôleur principal (102) selon l'une quelconque des revendications précédentes, dans lequel :
ledit outil d'entrée principal (106) comprend un dispositif de détection de force de préhension (125), adapté pour détecter l'action de pression de préhension exercée par les doigts (111, 112) du chirurgien déplaçant ledit premier corps allongé d'élément (114) et ledit deuxième corps allongé d'élément (116) l'un vers l'autre en dessous d'un angle de seuil de préhension prédéfini (γ) ; de préférence dans lequel
- lorsque l'action de pression de préhension exercée par les doigts (111, 112) du chirurgien rapproche ledit premier corps allongé d'élément (114) et ledit deuxième corps allongé d'élément (116) l'un de l'autre en dessous dudit angle de seuil de préhension prédéfini (γ), cela détermine une augmentation de la force de préhension appariée exercée par ledit dispositif de préhension chirurgical (117) ; de préférence dans lequel
ledit dispositif de détection de force de préhension (125) comprend au moins un ressort de préhension (130) sollicitant une extrémité libre d'élément déclencheur (129) d'un élément déclencheur (126) à l'écart dudit premier corps allongé d'élément (114), de sorte que ledit élément déclencheur (126) s'étend en porte-à-faux face audit deuxième corps allongé d'élément (116).

15. Ensemble contrôleur principal (102) selon l'une quelconque des revendications précédentes, dans lequel
- ledit ensemble contrôleur principal (102) comprend au moins un générateur de champ (107) générant un volume de champ prédéfini ; et dans lequel
- ledit ensemble capteur (119) détecte au moins la position dudit outil d'entrée principal (106) dans ledit volume de champ prédéfini.

16. Ensemble contrôleur principal (102) selon l'une quelconque des revendications précédentes,
dans lequel ledit ensemble capteur (119) comprend au moins un capteur d'articulation, de préférence un codeur, situé à l'intérieur de ladite articulation d'outil (118).

17. Ensemble contrôleur principal (102) selon l'une quelconque des revendications précédentes, dans lequel ledit ensemble capteur (119) détecte au moins la position mutuelle, et de préférence la position mutuelle et l'orientation relative, dudit premier corps allongé d'élément (114) et dudit deuxième corps allongé d'élément (116).

18. Système de chirurgie robotique (101) comprenant :
- au moins un ensemble contrôleur principal (102) selon l'une quelconque des revendications précédentes ;
- au moins un ensemble robot esclave (103), comprenant un instrument chirurgical esclave (104) conçu pour opérer sur l'anatomie d'un patient, ledit instrument chirurgical esclave (104) comprenant au moins un dispositif de préhension chirurgical (117) fournissant à l'instrument chirurgical esclave (104) un degré de liberté de mouvement de préhension ;
- une unité de commande (105), adaptée pour recevoir un premier signal de commande contenant des informations sur ladite commande manuelle et pour transmettre un deuxième signal de commande contenant des informations sur ladite commande manuelle à l'ensemble robot esclave (103) afin d'actionner ledit instrument chirurgical esclave (104).

19. Système de chirurgie robotique (101) selon la revendication 18, dans lequel ledit générateur de champ (107) est un générateur de champ magnétique.
